# EUROPEAN PATENT APPLICATION

(11) **EP 2 891 491 A1**
(43) Date of publication of application: **08.07.2015**
(21) Application number: 14000021.7
(22) Date of filing: 03.01.2014
(51) Int. Cl.: A61K 31/4015, C07D 201/00, A61K 31/165, A61K 31/198, A61K 31/485, A61K 31/496, A61P 43/00

(54) **Use of (r)-phenylpiracetam for the treatment of sleep disorders**

(71) Applicant: Merz Pharma GmbH & Co. KGaA, 60318 Frankfurt am Main (DE)
(72) Inventor: The designation of the inventor has not yet been filed
(74) Representative: Ricker, Mathias

(57) **Abstract**

The present invention relates to the efficient treatment of an individual afflicted with a sleep disorder, the instant treatment comprising administering to the individual an effective amount of (R)-phenylpiracetam or a pharmaceutically acceptable salt thereof.

## Description

### FIELD OF THE INVENTION

The present invention relates to the efficient treatment of an individual afflicted with a sleep disorder, the instant treatment comprising administering to the individual an effective amount of (R)-phenylpiracetam or a pharmaceutically acceptable salt thereof.

### BACKGROUND OF THE INVENTION

This invention relates to a method of treating patients afflicted with a sleep disorder, in particular excessive daytime sleepiness, particularly in narcolepsy or in Parkinson's Disease, or narcolepsy as such.

Excessive daytime sleepiness (EDS) is a state characterized by a persistent sleepiness, often in combination with a general lack of energy, even after having had apparently adequate, or even prolonged, duration of sleep in the night. EDS can be regarded as a broad condition encompassing a variety of disorders, where increased sleepiness is a symptom, or as a symptom of another underlying disorder like narcolepsy, obstructive sleep apnea or a circadian rhythm disorder.

Some persons with EDS, including those with hypersomnias like narcolepsy and idiopathic hypersomnia, are compelled to have a nap repeatedly during daytime; fighting off increasingly strong urges to sleep during inappropriate times such as while operating machines, such as driving cars, while at work, during a meal, or while being in conversations. As the compulsion to sleep gets stronger and stronger, the ability to complete tasks sharply diminishes, often mimicking the appearance of intoxication. During occasional unique and/or stimulating circumstances, a person with EDS can sometimes remain animated, awake and alert, for brief or extended periods of time. EDS can affect the ability to function in family, social, occupational, or other settings. The sleepiness may be so severe that patients with EDS can doze off with little warning; these episodes are commonly referred to as "sleep attacks". Extensive daily sleepiness usually improves temporarily after a brief nap, and most patients feel rested when they awake in the morning. A proper diagnosis of the underlying cause and ultimately treatment of symptoms and/or the underlying cause can help mitigate such complications. Patients can be scored using the Epworth Sleepiness Scale.

**Figure 1:**

| **Epworth Sleepiness Scale** | |
|---|---|
| How likely are you to doze off or fall asleep in the following situations in contrast to just feeling tired? This refers to your usual way in recent times. Even if you have not done some of these things recently, try to work out how they would have affected you. Use the following scale to choose the most appropriate number for each situation. | |
| 0 = would never doze | |
| 1 = slight chance of dozing | |
| 2 = moderate chance of dozing | |
| 3 = high chance of dozing | |

| **Situation** | **Chance of dozing** |
|---|---|
| Sitting and reading | |
| Watching TV | |
| Sitting inactive in a public place (eg, a theater or meeting) | |
| As passenger in a car for an hour without break | |
| Lying down to rest during the day when circumstances permit | |
| Sitting and talking to someone | |
| Sitting quietly after lunch without alcohol | |
| In a car, while stopped for a few minutes in traffic | |

| | |
|---|---|
| *Redrawn from Johns MW, Sleep. 1991;14(6): 540* | |

Narcolepsy is a clinical syndrome of daytime sleepiness with cataplexy, hypnagogic hallucinations, and sleep paralysis. It is the second most common cause of disabling daytime sleepiness after obstructive sleep apnea (Zeman et al, BMJ. 2004;329(7468):724; Scammell TE, Ann Neurol. 2003;53(2):154.

Narcolepsy with cataplexy is estimated to have a prevalence of 25 to 50 per 100,000 people and an incidence of 0.74 per 100,000 person-years (Longstreth WT et al., Sleep. 2007;30(1):13; Nohynek H et al., PLoS One. 2012;7(3):e33536. Epub 2012 Mar 28; Partinen M et al., PLoS One. 2012;7(3):e33723. Epub 2012 Mar 28). It is equally common in men and women (Ohayon MM et al, Neurology. 2002;58(12):1826; Coleman RM et al., JAMA. 1982;247(7):997; Silber MH et al., Sleep. 2002;25(2):197). Narcolepsy typically begins in the teens and early twenties, but may occur as early as at the age of five years, or as late as after 40 years of age. The prevalence of narcolepsy without cataplexy is uncertain, because it is not as well studied; however, it has been estimated to occur in 20 to 34 out of 100,000 people (Silber MH et al., Sleep. 2002;25(2):197; Shin YK et al., Acta Neurol Scand. 2008 Apr;117(4):273-8. Epub 2007 Oct 08).

Narcolepsy can be conceptualized as a disorder of sleep-wake control in which elements of sleep intrude into wakefulness and elements of wakefulness intrude into sleep. The result is the classic tetrad of chronic daytime sleepiness with varying amounts of cataplexy, hypnagogic hallucinations, and sleep paralysis. Only one-third of patients will have all of these classical findings; thus, the diagnosis of narcolepsy should be considered even among patients with chronic daytime sleepiness alone. The features of narcolepsy frequently worsen during the first few years and then persist for life (Landolfi JC and Nadkarni M, Neuro Oncol. 2003;5(3):214.

The diagnosis of narcolepsy is confirmed with a polysomnogram that rules out other sleep disorders and a Multiple Sleep Latency Test that demonstrates an average sleep latency of less than eight minutes and/or at least two sleep onset rapid eye movement periods (SOREMs). True cataplexy is highly suggestive of narcolepsy.

All patients with narcolepsy have chronic sleepiness, but they do not sleep more than healthy individuals during a 24 hour period (Dauvilliers Y et al., JAMA Neurol. 2013 Aug; Okun ML et al., Sleep. 2002;25(1):27. They are prone to fall asleep throughout the day, often at inappropriate times. Patients with narcolepsy typically have Epworth Sleepiness Scale scores >15.

Cataplexy is emotionally-triggered transient muscle weakness. Most episodes are triggered by strong, generally positive emotions such as laughter, joking, or excitement (Johns MW, Sleep. 1991;14(6):540. Episodes may also be triggered by anger or grief in some individuals. Cataplexy develops within three to five years of the onset of sleepiness in 60% of people with narcolepsy (Landolfi JC and Nadkarni M, Neuro Oncol. 2003;5(3):214).

The goals of therapeutic treatment of narcolepsy are to obtain "normal" alertness during conventional waking hours or to maximize alertness at important times of the day (eg, during work, school, or while driving). Once therapy has been optimized, the severity of residual sleepiness should be assessed with the Epworth Sleepiness Score or the Maintenance of Wakefulness Test, and persistently sleepy patients should be counseled to avoid potentially dangerous activities, such as driving cars.

Modafinil has become a first line pharmacologic therapy because it provides good control of sleepiness, is generally well tolerated, and illicit use is rare, as shown in several clinical trials (Randomized trial of modafinil for the treatment of pathological somnolence in narcolepsy. US Modafinil in Narcolepsy Multicenter Study Group, Ann Neurol. 1998;43(1):88; Randomized trial of modafinil as a treatment for the excessive daytime somnolence of narcolepsy: US Modafinil in Narcolepsy Multicenter Study Group. Neurology. 2000;54(5):1166). As a non-amphetamine "wakefulness promoting agent," its mechanism of action is not well understood, but it may increase dopaminergic signaling (Scammell TE et al., J Neurosci. 2000;20(22):8620; Wisor JP et al., J Neurosci. 2001;21(5):1787; Volkow ND et al., JAMA. 2009;301(11):1148).

Another drug that is used for the treatment of narcolepsy is methylphenidate, which is a central nervous system stimulant and a potent wakefulness-promoting drug. Although effective, it is considered a second-line agent because its sympathomimetic side effects can be problematic.

Like methylphenidate, amphetamines are central nervous system stimulants. Dextroamphetamine and methamphetamine may be the most potent wakefulness-promoting drugs, although some patients require relatively high doses. Other amphetamines include mixed amphetamine salts (Adderall) and lisdexamfetamine. The amphetamines are considered second-line agents because their sympathomimetic side effects can be problematic. By increasing aminergic signaling, these drugs can also improve cataplexy, hypnagogic hallucinations, and sleep paralysis (Mitler MM et al., Sleep. 1994;17(4):352; Mitler MM et al., J Clin Neurophysiol. 1990;7(1):93; Shindler J et al., Br Med J (Clin Res Ed). 1985;290(6476):1167).

Amphetamines can produce adverse effects similar to those seen with methylphenidate. These include cardiovascular events (eg, hypertension, sudden death) and psychiatric events (eg, psychosis, anorexia, addiction) (Nissen SE, N Engl J Med. 2006;354(14):1445; Mahowald MW and Bornemann MA, Sleep. 2005;28(6)). Patients prescribed amphetamines should be carefully informed about these risks and monitored regularly. These concerns are highlighted in new patient medication guides provided by the manufacturers of dextroamphetamine and methamphetamine.

Additionally, excessive daytime sleepiness (EDS) is common in patients with Parkinson's Disease (PD) (Hobson DE et al., JAMA 2002; 287:455; Gjerstad MD et al., Neurology 2006; 67:853). The sudden onset of sleep, a phenomenon known as sleep attacks, has also been described. EDS and sudden somnolence can be a hazard for PD patients when driving cars (Frucht S et al., Neurology 1999; 52:1908).

Management of EDS involves efforts to improve nocturnal sleep hygiene and to treat causes of poor nocturnal sleep. Pharmacologic treatment with modafinil, methylphenidate, or judicious use of coffee during the day was initially been regarded as potentially offering some benefit.

However, data regarding the effectiveness of modafinil are sparse and conflicting (Högl B et al., Sleep 2002; 25:905; Adler CH et al., Mov Disord 2003; 18:287; Ondo WG et al., J Neurol Neurosurg Psychiatry 2005, 76:1636). In the largest of these small trials, 40 subjects with PD and EDS were randomly assigned to treatment with modafinil (200 to 400 mg/day) or placebo (Ondo et al: J Neurol Neurosurg Psychiatry. 2005;76(12):1636). There was no benefit for modafinil treatment in the primary outcome measure, the Epworth sleepiness score, or any of the secondary outcome measures. Modafinil is not approved by the US Food and Drug Administration (FDA) for the treatment of sleepiness in PD. Thus, based on these data it appears to be the case that EDS in PD is based on a particular mechanism different from EDS in non-PD patients.

Thus, despite the fact that many attempts have been made to develop agents for the treatment of sleep disorders, in particular EDS, particularly in Parkinson's Disease, and narcolepsy, so far these attempts have had limited clinically meaningful success in patients.

Thus, there is still a large unmet need to identify medicaments for improving the treatment of sleep disorders.

The solution provided by the present invention to solve this problem, i.e. the use of a particular compound, has so far not been achieved or suggested by the prior art.

### SUMMARY OF THE INVENTION

The present invention relates to a method of improving the sleep disorders, in particular EDS, particularly in narcolepsy or in Parkinson's Disease, and narcolepsy as such, in a subject in need thereof, comprising the step of administering an effective amount of (R)-phenylpiracetam or a pharmaceutically acceptable salt thereof.

Thus, the present invention relates to a pharmaceutical composition comprising (R)-phenylpiracetam or a pharmaceutically acceptable salt thereof for use in the treatment of a sleep disorder.

### BRIEF DESCRIPTION OF THE DRAWINGS

**Figure 1** shows that (R)-phenylpiracetam increases extracellular dopamine levels in rat striatum as shown by brain microdialysis.
**Figure 2** shows the concentration of (R)-phenylpiracetam in the brain, as determined by brain microdialysis, after intraperitoneal (i.p.) application. Affinity for DA transporter is c.a. 13 µM.
**Figure 3** shows that (R)-phenylpiracetam increases locomotor activity (horizontal activity) in rats (administration i.p. 15 min before test) (*: p< 0.05 vs. vehicle, Kruskal-Wallis one-way ANOVA on ranks at each time interval followed by rank sum test).
**Figure 4** shows the CNS profile of (R)-phenylpiracetam in an electroencephalography (EEG) screen indicating activatory effect on dopaminergic system.
**Figure 5** shows the results from a progressive ratio test for determining the influence of (R)-phenylpiracetam on motivation in comparison to amphetamine.
**Figure 6** shows the results from a progressive ratio test for determining the influence of (R)-phenylpiracetam on motivation in comparison to methylphenidate.
**Figure 7** shows the results from a cost benefit test for determining the influence of (R)-phenylpiracetam on motivation in comparison to amphetamine.
**Figure 8** shows the results from a cost benefit test for determining the influence of (R)-phenylpiracetam on motivation in comparison to methylphenidate.

### DETAILED DESCRIPTION OF THE INVENTION

The peculiarity of this invention compared to former treatment approaches for treating sleep disorders is the so far unknown therapeutic efficacy of (R)-phenylpiracetam, which is presumably based at least in part on the newly identified activity of (R)-phenylpiracetam as the dopamine re-uptake inhibitor (see Example 1).

Thus, the present invention relates to the use of (R)-phenylpiracetam and any of its salts, solvates and conjugates, which possesses at least an inhibitory activity on the dopamine re-uptake transporter.

Thus, in a first aspect the present invention relates to a pharmaceutical composition comprising (R)-phenylpiracetam or a pharmaceutically acceptable salt thereof for use in the treatment of a sleep disorder.

The term "phenylpiracetam" is known in the art and refers to the compound 2-(4-phenyl-2-oxopyrrolidin-1-yl)acetamide (C₁₂H₁₄N₂O₂; MW 218.3 g/mol). Phenylpiracetam is also known as fenotropil, phenotropyl, phenotropil, fenotropyl, or carphedon and was developed in Russia, where it is available as a prescription medicine under the name "Phenotropil^{®}". As used herein, phenylpiracetam refers to the substance, as well as its pharmaceutically acceptable salts.

Phenylpiracetam was developed in Russia, where it is available as a prescription medicine under the name "Phenotropil®". Several studies about the effect of phenylpiracetam in various diseases and disorders, including in the treatment of Parkinson's Disease, have been performed in Russia.

Phenylpiracetam is optically active and is available as a racemate of the two enantiomers. The International Nonproprietary Name (INN) "Fonturacetam" has been assigned to racemic phenylpiracetam. An international patent application with priority date in 2006 first disclosed the separation of the two enantiomers and demonstrated that the (R)-enantiomer is predominantly responsible for the pharmacological activity (WO 2007/104780). (R)-phenylpiracetam showed more pronounced activity in animal models for detecting antidepressant, analgesic, muscle relaxant and psychostimulant effects. The patent application claims (R)-phenylpiracetam for the use as an antidepressant, as a stress-protective agent, as a modulator of locomotor activity, as a muscle relaxant and as an analgesic.

More detailed pharmacological data of (R)-phenylpiracetam have been published recently (Zvejniece et al., Basic Clin Pharmacol Toxicol. 109, 407-412, 2011). In the open-field test a significant increase in locomotor activity was found, which was just slightly stronger with the (R)-phenylpiracetam than with (S)-phenylpiracetam. Also in the forced swim test, used as a model for depression, (R)-phenylpiracetam was also only slightly more potent than (S)-phenylpiracetam. However, in the passive avoidance test (R)-phenylpiracetam enhanced memory function significantly better compared to (S)-phenylpiracetam. The authors conclude that these results may be important for the clinical use of optically pure isomers of phenylpiracetam.

Prior to the surprising finding of the present inventors that (R)-phenylpiracetam acts as a dopamine re-uptake transporter inhibitor, the precise pharmacological mechanism of action of (R)-phenylpiracetam had not been elucidated. In pharmacological studies phenylpiracetam was found to activate the operant behaviour, to counteract psychodepressant effects of diazepam, to inhibit post-rotational nystagmus, and to prevent the development of retrograde amnesia. It also exhibited anticonvulsant action (Bobkov et al., Biull Eksp Biol Med. 95, 50-53, 1983) and some neuroprotective activity in experimental cerebral ischemia (Tiurenkov et al., Eksp Klin Farmakol. 70, 24-29, 2007). As described in the current application, (R)-phenylpiracetam enhances progressive ratio responding much stronger than methylphenidate which is a DAT inhibitor. Thus, phenylpiracetam exhibits additional or different pharmacological effects, which are not yet fully identified and are differentiating phenylpiracetam from other pure dopamine re-uptake transporter inhibitors.

In humans phenylpiracetam is administered orally and shows a half-life of 3-5 hours. There are only a small number of low-scale exploratory clinical trials predominantly published in Russian journals. They have shown possible links between intake of phenylpiracetam and improvement in a number of conditions and diseases including asthenic syndrome and autonomic disturbances in brain trauma (Kalinskij and Nazarov, Zh Nevrol Psikhiatr Im S S Korsakova.107, 61-63, 2007), brain organic lesions (Savchenko et al. Zh Nevrol Psikhiatr Im S S Korsakova.105, 22-26, 2005), epilepsy (Bel'skaia et al. Zh Nevrol Psikhiatr Im S S Korsakova.107, 40-43, 2007, Lybzikova et al., Zh Nevrol Psikhiatr Im S S Korsakova.108, 69-70, 2008), stomatological problems (Novikova et al. Stomatologiia (Mosk).87, 41-45, 2008), and vascular encephalopathy (Gustov et al. Zh Nevrol Psikhiatr Im S S Korsakova.106, 52-53, 2006). Effects of phenylpiracetam on immunological consequences of stroke have also been described (Gerasimova et al., Zh Nevrol Psikhiatr Im S S Korsakova.105, 63-64, 2005).

According to the package insert of the drug as approved in Russia, phenylpiracetam is a nootropic drug, which has an expressed anti-amnesic action, a direct activating effect on the integrative activity of the brain, helps consolidate memory, improves concentration and mental performance, facilitates the learning process, increases the information transfer between the hemispheres of the brain, increases the resistance of brain tissue to hypoxia and toxic effects, has anticonvulsant and anxiolytic effects, regulates the processes of activation and inhibition of central nervous system, and improves mood. It is furthermore stated that phenylpiracetam has a positive effect on the metabolism and blood circulation in brain, stimulates the redox processes and increases energy potential through utilization of glucose, improves regional blood flow in ischemic areas of the brain. It increases noradrenaline, dopamine and serotonin content in the brain, does not affect the levels of GABA, associates neither with GABAA nor GABAB receptors, has no noticeable effect on the spontaneous bioelectric activity of the brain, does not influence respiration and the cardiovascular system. It shows no significant diuretic effect and has anorexigenic effect during treatment. According to the Russian package insert, the stimulating effect of phenylpiracetam manifests in the ability to provide a moderate effect on motor responses, to enhance physical performance. The moderate psychostimulant effect of the drug is combined with an anxiolytic activity, and it improves mood, has some analgesic effect and raises the threshold of pain. The adaptogenic effect of phenylpiracetam is manifested in increasing resistance to stress in conditions of excessive mental and physical overload, fatigue, hypokinesia and immobilization, and at low temperatures.

While (R)-phenylpiracetam was synthesized and described in the literature as nootropic compound, the present applicants have surprisingly found that (R)-phenylpiracetam has features not fitting into the class of nootropics.

The term nootropics was created in 1960s, with the discovery of piracetam followed by pramiracetam, oxiracetam, aniracetam, tenilsetam and others, and is nowadays often used to describe cognitive enhancers that are neuroprotective and nontoxic. Most nootropics do not have a clearly defined mechanism of action and work, and seem to exert their cognitive and/or neuroprotective effects through changes in brain blood flow, energy management etc.

In contrast to nootropics, (R)-phenylpiracetam appears to have a clearly defined mechanism of action by inhibition of dopamine (DA) uptake, and behavioural effects are seen at the doses that create in the brain levels within affinity for DA uptake seen *in vitro* and produce an increase in the DA concentration in the brain as assessed by brain microdialysis (20 µM at 50 mg/kg with an affinity constant of 13 µM; see Examples).

Furthermore, in the EEG pattern there is a clear difference between nootropics and (R)-phenylpiracetam. For nootropics, a decrease of power for nearly all waves can be shown in the reticular formation, while there is no change of theta, delta, alpha1 and beta2 waves for (R)-phenylpiracetam (see Examples).

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the federal or a state government or listed in the U.S. Pharmacopeia or other generally recognized pharmacopeia for use in mammals, and more particularly in humans.

The term "salt" is defined as a chemical containing different charged components. The term salt also includes hydrates and solvates.

(R)-Phenylpiracetam may be used according to the invention in the form of any of pharmaceutically acceptable salts, solvates and conjugates. Any references to (R)-phenylpiracetam in this description should be understood as also referring to such salts, solvates and conjugates.

The term "treat" is used herein to mean to relieve or alleviate at least one symptom of a disease in a subject. Within the meaning of the present invention, the term "treat" also denotes to arrest, delay the onset (*i.e*., the period prior to clinical manifestation of a disease) and/or reduce the risk of developing or worsening a disease. Thus, "treatment" as used herein includes modifying, curative and symptomatic treatments.

As used herein, the term "subject" encompasses mammals including animals and humans.

In particular embodiments, said sleep disorder is excessive daytime sleepiness, particularly excessive daytime sleepiness in narcolepsy or in Parkinson's Disease.

In particular embodiments, said sleep disorder is narcolepsy.

In particular embodiments, said sleep disorder is excessive daytime sleepiness in patients with Parkinson's Disease

In particular such embodiments, said sleep disorder is not a sleep disorder caused by D2-dopamine receptor agonists in Parkinson's Disease.

In particular other such embodiments, said sleep disorder is not a sleep disorder caused by agents for dopamine-replacement therapy in Parkinson's Disease.

In particular other embodiments, said sleep disorder is not a sleep disorder in patients with Parkinson's Disease.

In particular embodiments, (R)-phenylpiracetam is present as (R)-phenylpiracetam hydrochloride.

In particular embodiments, (R)-phenylpiracetam is for administration in a range from about 1 mg to about 250 mg/day, or in a range from about 25 mg to about 200 mg/day, or in a range from about 50 mg to about 150 mg/day.

In particular embodiments, (R)-phenylpiracetam or a pharmaceutically acceptable salt thereof is for administration once a day, twice a day, or three times a day.

In particular embodiments, (R)-phenylpiracetam or a pharmaceutically acceptable salt thereof is for administration in an oral formulation.

In particular embodiments, (R)-phenylpiracetam or a pharmaceutically acceptable salt thereof is for administration in combination with at least one additional pharmaceutical agent which has been shown to be effective for the treatment of said sleep disorder.

### EXAMPLES

The following example illustrates the invention without limiting its scope.

### EXAMPLE 1: Determination of (R)-phenylpiracetam targets

Using functional monoamine transporter assays, we determined the potential drug targets for (R)-phenylpiracetam.

Recombinant Chinese hamster ovary (CHO) cells CHO-K1 (ATCC^{®} CCL-61 ™) cells stably expressing dopamine transporters are plated. The cells (2 x 10⁵/ml) are pre-incubated with test compound and/or vehicle in modified Tris-HEPES buffer pH 7.1 at 25°C for 20 min and 50 nM [³H]-dopamine is then added for an additional 15 min incubation period. Non-specific signal is determined in the presence of 10 µM nomifensine. Cells are then solubilized with 1% SDS lysis buffer. Reduction of [³H]-dopamine uptake by 50 percent or more (≥50%) relative to vehicle controls indicates significant inhibitory activity. Compounds are screened at 10, 1, 0.1, 0.01 and 0.001 µM. These same concentrations are concurrently applied to a separate group of untreated cells and evaluated for possible compound-induced cytotoxicity only if significant inhibition of uptake is observed.

Recombinant Madin Darby canine kidney (MDCK) cells (NBL-2) (ATCC^{®} CCL-34™) expressing norepinephrine transporter are plated for two days. Test compound and/or vehicle is pre-incubated with cells (1 x 10⁵/ml) in modified Tris-HEPES buffer pH 7.1 for 20 min at 25°C and 25 nM [³H]-norepinephrine is then added for an additional 15 min incubation period. A lysate is obtained from solubilized cells and counted to determine [³H]-norepinephrine uptake. Reduction of [³H]-norepinephrine uptake by 50 percent or more (≥50%) relative to 10 µM desipramine indicates significant inhibitory activity. Compounds are screened at 10, 1, 0.1, 0.01 and 0.001 µM. These same concentrations are concurrently applied to a separate group of untreated cells and evaluated for possible compound-induced cytotoxicity only if significant inhibition of uptake is observed.

These pharmacological experiments showed an affinity of (R)-phenylpiracetam for the neuronal dopamine re-uptake transporter of 13 µM in functional assays.

In microdialysis experiments, we could show that at a behaviorally active dose of 100 mg/kg a free concentration 50 µM is reached in extracellular fluid in the brain, and at 50 mg/kg a free concentration of 20 µM.

These concentrations are sufficiently high to postulate that the dopamine re-uptake transporter is a relevant target in the brain.

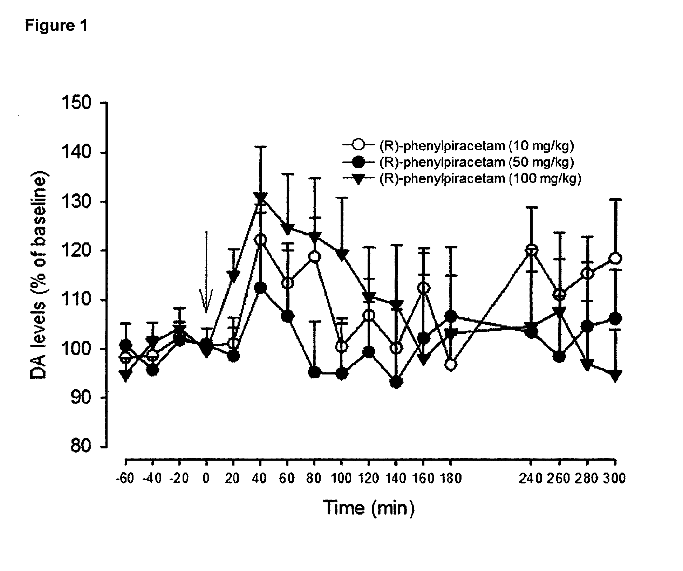

### EXAMPLE 2: Assessment of (R)-phenylpiracetam on extracellular dopamine levels in rat striatum using brain microdialysis - Fig. 1

### Test Item

(R)-Phenylpiracetam was dissolved in saline and injected intraperitoneally (i.p).

### Animals

Adult male Sprague-Dawley rats (n = 5, ∼300 g; Harlan, The Netherlands) were used for the experiments. After surgery animals were housed individually (cages 30 cm x 30 cm x 40 cm) with food and water was *ad libitum* available at standard conditions. The post-surgery interval for recovery was minimally 48 h.

### Surgery

Rats were anesthetized using isoflurane (2%, 800 ml/min O₂), and placed in a stereotaxic frame (Kopf instruments, USA). I-shaped probes (Hospal AN 69 membrane, 3 mm exposed surface; Brainlink, the Netherlands) were inserted into striatum. Coordinates for the tips of the probes were: posterior (AP) = + 0.9 mm to bregma, lateral (L) = +3.0 mm to midline and ventral (V) = -6.5 mm to dura (Paxinos and Watson, 1982).

### Microdialysis experiments

Experiments were performed 24-48 hours after surgery. On the day of the experiment, the probes were connected with flexible polyether ether ketone (PEEK) tubing to a microperfusion pump (Syringe pump UV 8301501, Univentor, Malta) and perfused with artificial cerebro-spinal fluid (aCSF), containing 147 mM NaCl, 3.0 mM KCI, 1.2 mM CaCl₂, and 1.2 mM MgCl₂, at a flow rate of 1.5 µl/min. Microdialysis samples were collected for 20 minute periods up to 120 min using automated fraction collector (Univentor 820 Microsampler, Antec, Netherlands), and stored at -80°C pending analysis. After the experiment, the rats were sacrificed and the brains were removed. The position of each probe was histologically verified according to Paxinos and Watson (1982) by making coronal sections of the brain.

### Determination of Dopamine (DA) and 3,4-Dihydroxyphenylacetic acid (DOPAC)

### Separation of DA and DOPAC

Samples (20 µl) were injected onto the high-performance liquid chromatography (HPLC) column (Reversed Phase, particle size 3 µm, C18, Thermo BDS Hypersil column, 150 mm x 2.1 mm, Thermo Scientific, USA) by a refrigerated microsampler system, consisting of a syringe pump (Gilson, model 402, France), a multi-column injector (Gilson, model 233 XL, France), and a temperature regulator (Gilson, model 832, France). Chromatographic separation was performed using a mobile phase that consisted of a NaAc buffer (6.15 g/I) with methyl alcohol (2.5% v/v), Titriplex (250 mg/l), 1-octanesulfonic acid (OSA, 150 mg/l), and adjusted with glacial acetic acid to pH 4.1 (isocratic). The mobile phase was run through the system at a flow rate of 0.35 ml/min by an HPLC pump (Shimadzu, model LC-10AD vp, Japan).

### Detection

Concentrations of DA and DOPAC were determined in the same sample, by HPLC separation and electrochemical detection. DA and DOPAC were detected electrochemically using a potentiostate (Antec Leyden, model Intro, the Netherlands) fitted with a glassy carbon electrode set at +500 mV vs. Ag/AgCl (Antec Leyden, the Netherlands). Data was analyzed by Chromatography Data System (Shimadzu, class-vp, Japan) software. Concentrations were quantified by the external standard method.

### Statistical Analysis

After transmitter levels were stabilized, four consecutive pre-treatment microdialysis samples with less than 50% variation were taken as baseline and their mean was set at 100%. After compound 3-2008 (100 mg/kg) was administered 13 more fractions were taken. Transmitter concentration in each fraction was expressed as % ± SEM (standard error mean) of baseline.

### Results

The results show that (R)-phenylpiracetam increases concentration of dopamine in the striatum.

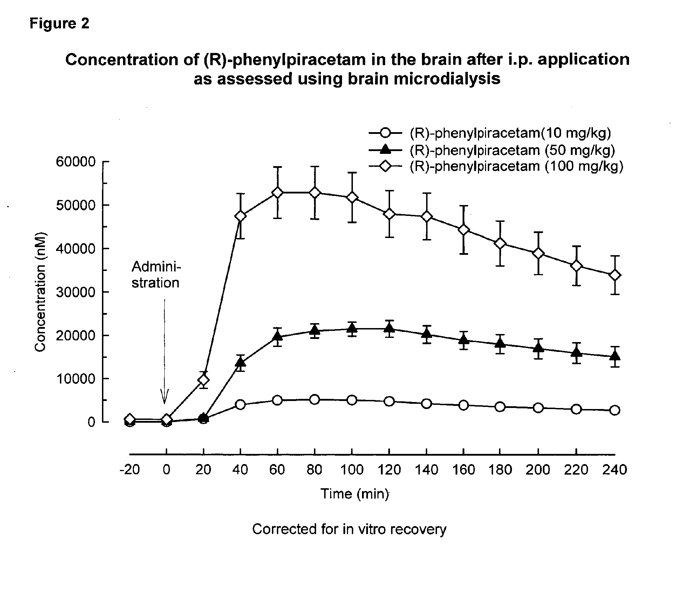

### EXAMPLE 3: Assessment of the concentration of (R)-phenylpiracetam in the brain using brain microdialysis - Figs. 2, 14, 15

### Subjects

Naive adult male Sprague-Dawley rats (240-360 g, Janvier, France) were used for the study kept under standard conditions. All experiments were conducted during the light period of the day-night cycle.

### Surgery

Siliconized guide cannula (MAB 6.14.IC) (MAB, Stockhom, Sweden) were implanted unilaterally in pentobarbital anaesthetized animals aiming at the *caudatus putamen* (CPu; AP: +0.1, LM: ± 2.6, DV: -3.2 mm relative to bregma; -3.3 mm interaural) according to the atlas of Paxinos and Watson (loc. cit.). Rats were given at least 3 days to recover from surgery before starting microdialysis experiments.

### Microdialysis

Microdialysis experiments were performed in the home cage of the animal. A microdialysis probe (MAB 6.14.4.; 4 mm exposed membrane length, polyethersulfone (PES) membrane; MAB, Stockholm, Sweden) was lowered through the guide cannula into the CPu (ventral position of probe tip with reference to the skull: -7.2 mm) ca. 12 hours before the sampling and left in place for the whole testing period.

The probes were perfused with aCSF at a flow rate of 2 µl/min using a CMA 102 perfusion pump (CMA, Solna, Sweden). The composition of the aCSF was 147 mM Na⁺, 2.7 mM K⁺, 1.2 mM Ca²⁺, 0.85 mM Mg²⁺, 0.04 mM ascorbic acid. The animals were connected by a head block tether system (Instech, Plymouth Meeting, USA) to a dual channel liquid swivel 375/D/22QM (Instech, Plymouth Meeting, USA). Fluorinated ethylene propylene (FEP) tubing and tubing adapters (MAB, Stockholm, Sweden) were used. The sample collection began one hour after start of perfusion with three 20-minutes fractions (baseline). Thereafter, each rat was injected i.p. with (R)-phenylpiracetam. The samples (40 µl) were collected automatically with a fraction collector (CMA/142; CMA, Solna, Sweden) and stored at -20°C until analysis.

### Determination of recovery

To perform *in vitro* recovery, probes were inserted into a beaker with aCSF (37°C) containing 100 nM solution of (R)-phenylpiracetam or L-DOPA. Five samples (40 µl) were collected while only the last 2 were used for analysis of (R)-phenylpiracetam or L-DOPA concentration.

### Analytics

HPLC in combination with atmospheric pressure ionization tandem mass spectrometry (API-MS/MS) was employed (HPLC (Shimadzu Prominence, Duisburg, Germany) coupled to an API 4000 Q Trap (triple quadrupole, Applied Biosystems/MDS Sciex, Darmstadt, Germany) equipped with a Turbolonspray source (ESI)). The analytical column was an Onyx Monolithic C18 50 mm x 2 mm (Phenomenex, Aschaffenburg, Germany). The mobile phase consisted of the eluent A water and eluent B acetonitrile both containing 0.1 % of formic acid. The chromatographic run consisted of a gradient over 1.5 min from 5% acetonitrile in water at start until a mobile phase composition of 50 % acetonitrile in water. A volume of 1 ml was injected in the API/MS/MS. A standard curve was used to calculate sample concentrations of studied agents in our samples.

### Results

The results show that (R)-phenylpiracetam reaches brain levels sufficient to affect primary target (DA carrier). This shows that interactions observed in behavioural experiments are of pharmacodynamic, not pharmacokinetic nature.

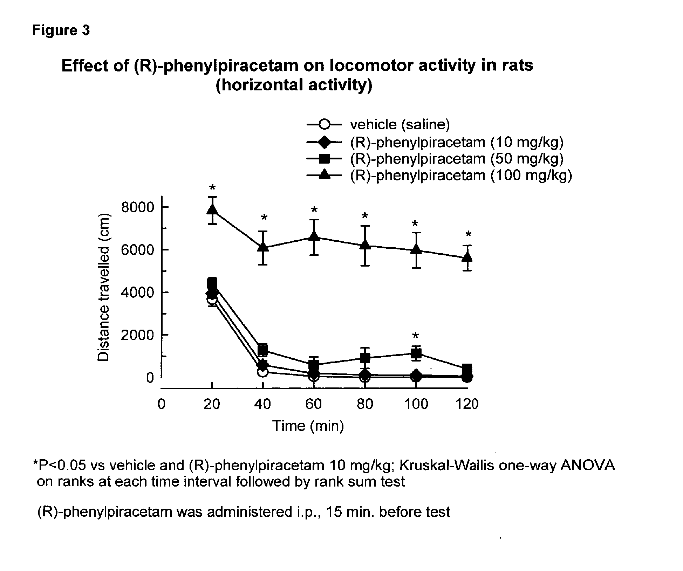

### EXAMPLE 4: Effect of (R)-phenylpiracetam on locomotor activity (horizontal activity) in rats - Fig. 3

### Animals

Experimentally naive adult male Sprague-Dawley rats (230-300 g) are kept four per cage, in a room with controlled temperature (21 ± 1°C) and humidity. Food and water are available *ad libitum* and the animals are kept under an alternating 12 h / 12 h day-night cycle (lights on at 07.00) for at least 6 days before the experiments are started. Each animal is used only once. Experimental group consist of 8 animals per group.

### Procedure

Horizontal locomotor activity was measured in 8 perspex boxes (ENV-515-16, 43.2 cm x 43.2 cm x 30 cm), Med-Associates Inc.) equipped with 4 arrays of 16 infrared photobeams placed 3 cm above the floor. Distance travelled (DT) was used in further analysis as a measure of locomotion.

### Treatment

(R)-Phenylpiracetam was administered i. p. in a volume of 2 ml/kg in saline

### Statistical analysis

Data with locomotor activity were analyzed by means of Kruskal-Wallis ANOVA on ranks followed, if significant, by rank sum test.

### Results

Figure 3 shows that (R)-phenylpiracetam increases locomotor activity (horizontal activity) in rats starting at the dose of 100 mg/kg indicating stimulatory activity. **[NOTE: can we add a statement that this activity is indicative of potential efficacy in sleep disorders]**

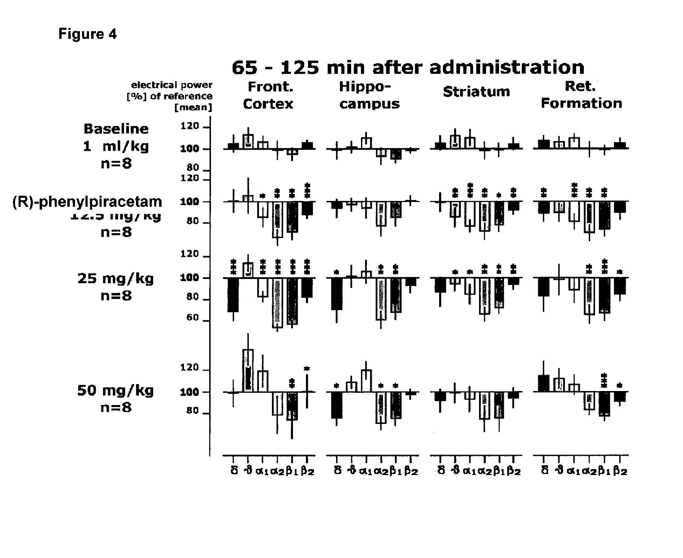

### EXAMPLE 5: Testing of (R)-phenylpiracetam in an electroencephaloaraphy (EEG) screen - Fig. 4

(R)-Phenylpiracetam was tested at four different concentrations (1 mg/kg, 12.5 mg/kg, 25 mg/kg, and 50 mg/kg) in an EEG screen as described by Dimpfel (Dimpfel, Neuropsychobiology, 58 (2008)178-86).

### Animals

Eight adult Fisher 344 rats (8 months of age, kept on on inverse light-dark cycle, weight about 400 g, provided by Charles River Laboratories, D-97633, Sulzfeld) were used in this experimental series. Animals were implanted with electrodes into the brain and were given two weeks for recovery from surgery. After this the transmitter was plugged in for adaptation and control experiments. During the recording rats were not restricted and could move freely but did not have food available (chewing would have produced too many artifacts).

### Acclimatisation and housing conditions

The animals were allowed to acclimatize for at least 4 weeks before the study started. There was automatic control of light cycle, temperature and humidity. Light hours were 18 h in the evening - 6 h in the morning. Daily monitoring indicated that temperature and humidity remained within the target ranges of 22 ± 2°C and 44 ± 5% respectively. Cages, bedding, and water bottles were changed at regular intervals, *i.e.* every 2-3 days. Standard diet (Nohrlin H10, Altromin, D-32791 Lage, Germany) was available to the animals *ad libitum.* The animals had access to domestic quality mains water *ad libitum.*

### Surgery

Rats were implanted with 4 bipolar concentric steel electrodes within a stereotactic surgical procedure during anaesthesia with ketamine. All four electrodes were placed 3 mm lateral within the left hemisphere. Dorsoventral coordinates were 4, 6, 4.2 and 8 mm and anterior coordinates were 3.7, 9.7, 5.7 and 12.2 mm for frontal cortex, striatum, hippocampus, and reticular formation, respectively (according to the atlas of Paxinos and Watson, 1982). A pre-constructed base plate carrying 4 bipolar stainless steel semi-micro electrodes (neurological electrodes "SNF 100" from Rhodes Medical Instruments, Inc., Summerland, CA 93067, USA) and a 5-pin-plug was fixed to the skull by dental cement interacting with 3 steel screws placed on distance into the bone. The distant recording spot of the electrode was the active electrode whereas the proximal spots of the four electrodes were connected to each other to give a reference. The base plate was carrying a plug to receive later on the transmitter (weight: 5.2 g including battery, 26 mm x 12 mm x 6 mm of size).

### Experimental Procedure

EEG signals were recorded from frontal cortex, hippocampus, striatum and reticular formation of freely moving rats from inside a totally copper shielded room. Signals were wirelessly transmitted by a radio-telemetric system (Rhema Labortechnik, Hofheim, Germany, using 40 MHz as carrier frequency) and were amplified and processed as described earlier to give power spectra of 0.25 Hz resolution (Dimpfel et al. 1986; Dimpfel et al. 1988; Dimpfel et al. 1989; Dimpfel, 2003). In short, after automatic artifact rejection signals were collected in sweeps of 4 s duration and fast-fourier transformed using a Hanning window. Sampling frequency was 512 Hz. Four values were averaged to give a final sampling frequency of 128 Hz, well above the Nyquist frequency. The resulting electrical power spectra were divided into 6 specially defined frequency ranges (delta: 0.8 - 4.5 Hz; theta: 4.75 - 6.75 Hz; alpha1: 7.00 - 9.50 Hz; alpha2: 9.75 - 12.50 Hz; beta1: 12.75 - 18.50 Hz; beta2: 18.75 - 35.00 Hz). Spectra were averaged in steps of 3 min each and displayed on-line. In an off-line procedure spectra were averaged to give longer periods for further analysis and data presentation.

### Test Items

(R)-Phenylpiracetam was injected i.p. in saline followed by recording of "Tele-Stereo-EEG" intracerebral field potentials in combination with a video tracking system for detection of changes in motility (GJB Datentechnik GmbH, D-98704 Langewiesen, Germany). This system recognized locomotion as well as stereotyped behaviour by following a contrast difference of the black transmitter on the head of the animal in comparison to its environment. The system has been validated in a previous study with different dosages of caffeine.

### Treatment Groups

After surgery all animals were randomly allocated to treatment groups, such that the treatment groups were evenly distributed throughout the caging system. A crossover design with at least one week of drug holidays in between the administrations was used. After a pre-drug period of 45 min for pre-drug recording, drug effects were observed continuously on the screen (artifact control) for 300 min subdivided into 15 min periods after a lag time of 5 min for calming of animals after i.p. administration. Changes of electrical power are expressed as % of the 45 min absolute pre-drug spectral power values within each frequency band.

### Statistical Analysis

Data are expressed as mean values ± S.E.M. Statistics were calculated by means of the Wilcoxon-Mann-Whitney U-test for comparison to results obtained by vehicle injection at the particular time frame. For comparison of data to reference compounds tested earlier under identical conditions discriminant analysis according to Fischer was used. A total of 24 variables (six frequency ranges times 4 brain areas) were used for analysis. Firstly, spherical projection of the results from 47 reference compounds plus physiological sleep was performed using the three spatial coordinates for the first three discriminant axes. Secondly, coding of the result of the fourth to sixth discriminant analysis into red, green and blue was followed by an additive color mixture in analogy to the so-called RGB mode (as used in TV). This matrix of drug actions is kept constant (frozen) for classification of unknown preparations since addition of further compounds would otherwise change the projections.

### Results

(R)-Phenylpiracetam produced a dose dependent attenuation of alpha2 and beta1 waves. The highest dosage produced a different pattern of changes in that theta power increases within the frontal cortex were observed. Motility was increased. Alpha2 waves are mainly under the control of dopamine (Dimpfel, loc. cit.). Thus, direct or indirect effects on dopaminergic neurotransmission can be expected from (R)-phenylpiracetam. Attenuation of alpha2 and beta1 waves has also been observed after administration drugs used for treatment of M. Parkinson (Dimpfel and Hoffmann, Neuropsychobiology, 62, 213-20, 2010). In summary (R)-phenylpiracetam shows a profile within the area of stimulatory drugs and in turn would be expected to give benefit in excessive sleep disorders.

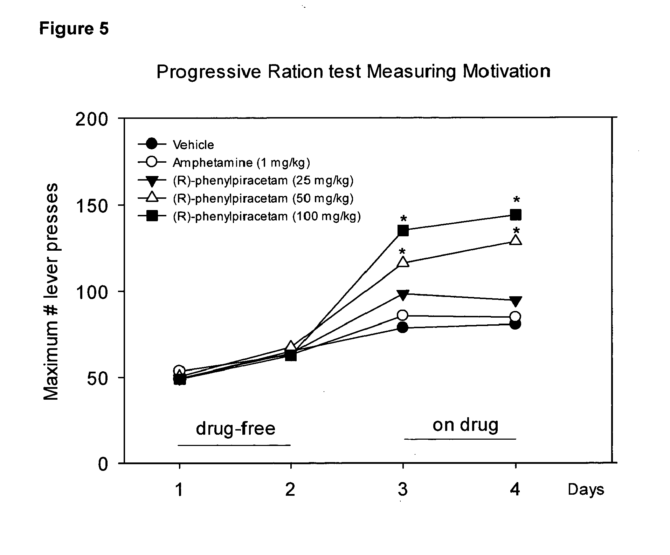

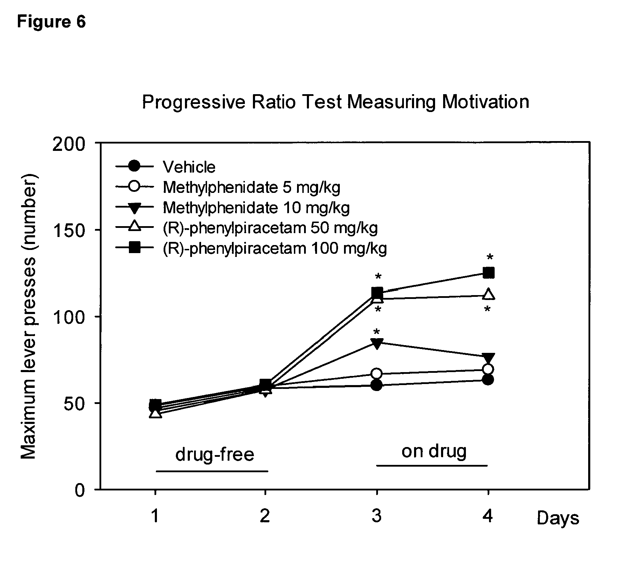

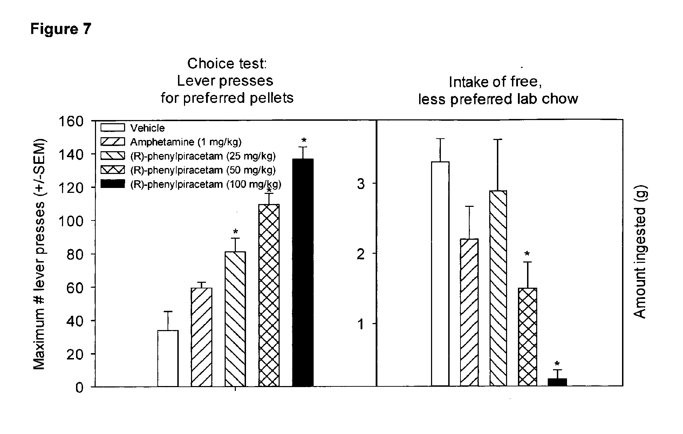

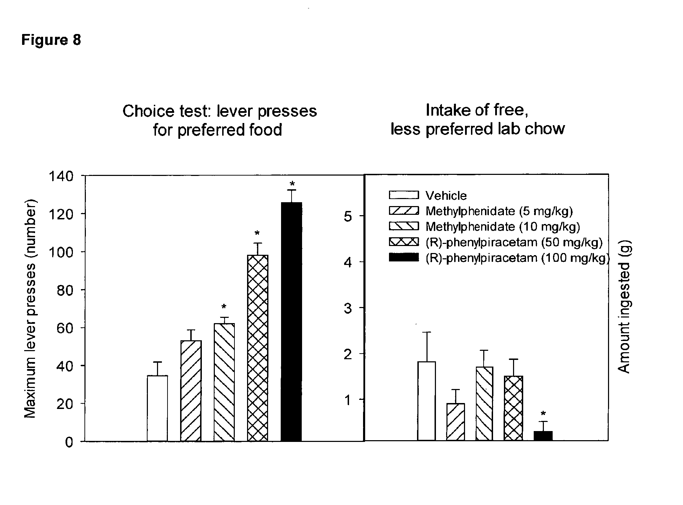

### EXAMPLE 6: Effect of (R)-phenylpiracetam in motivation tests - Figs. 5, 6, 7, 8

### Test Item

Methylphenidate purchased from Sigma (Taufkirchen, Germany) was dissolved in distilled water fresh for each test day. D-Amphetamine purchased from Sigma (Taufkirchen, Germany) was dissolved in 0.9% sterile NaCl solution fresh for each test day. (R)-Phenylpiracetam was dissolved in distilled water fresh for each test day.

### Animals

One hundred and three male Sprague-Dawley rats (Janvier, Le Genest-St-Isle, France) weighing 225-250 g on arrival were used.

### Acclimatisation and housing conditions

The animals were kept under standard laboratory conditions in groups of up to 4 per care with *ad libitum* access to water. Standard diet for rodents (Altromin) was available to the animals at 15 g per animal/day

### Treatment Groups

For each test described below, two independent experiments were performed using an identical protocol. After continuous reinforcement training (CRF) training (see below) all animals were assigned to treatment groups, such that performance across all the groups was similar. The treatment groups were evenly distributed throughout the caging system.

### Experimental methods

In experiment 1 and 2 the following protocols were used:

Overview: Three tests were performed in a consecutive manner on subsequent days. On day 1-2, animals were habituated individually to Skinner boxes in two sessions, one per day, for 30 min. Animals received "free" food pellets at a random time (one pellet per minute on average). On days 3-8 animals were trained in Skinner boxes on a continuous reinforcement schedule (one pellet per lever press). On days 9-10 they were tested in the progressive ratio test (Test 1) off-drug, on days 11-12 on-drug. On day 13, a choice test (Test 2) was performed on-drug, on day 14, a consumption test (Test 3) was performed on-drug. Below, tests are described in detail.

### Test 1: Progressive ratio (PR) task

Behavioral testing was conducted in 12 operant test chambers (Med Associates, St. Albans, USA). Each chamber was equipped with a retractable lever, a food dispenser with receptacle, an overhead house light and two stimulus lights, one above the lever and the other above the food receptacle. An infrared photocell beam detected nose pokes into the food receptacle. The apparatus was controlled by a computer system (SmartControl®-Interface and MedPC-software, Med Associates, St. Albans, USA). The light above the food receptacle indicated the delivery of a food pellet in the receptacle. Rats were first habituated to the operant boxes for two sessions (30 min each) on two consecutive days. Thereafter, animals were trained for six sessions on the CRF schedule for 30 min. Followed by 4 sessions (two session off-drug followed by two sessions on-drug) of an increasing fixed ratio (FR) schedule in steps of 5, with 3 repetitions of each step (*i.e.* 1-1-1; 5-5-5; 10-10-10; ...). When the required FR value of each trial was achieved the light above the food receptacle indicated the delivery of a single food pellet (45 mg, Bioserve, USA). The light remained on until the rat poked its nose in the receptacle. Lever presses while the light was on were counted as perseverative lever presses; they were counted but had no programmed consequences. A session lasted for 90 min or was ended when a rat failed to press the lever for consecutive 10 min. For each session, the value of the last completed ratio (breaking point) was recorded as well as the amount of received rewards, the perseverative lever presses, the duration of the session and the latency to respond. Animals of all treatment groups received respective vehicle/drug infusions 30 min pre-test on the final two sessions involving testing under a PR schedule.

### Test 2: Cost-benefit choice test

In this task the rats had the choice between working for their preferred food (Bioserve pellets) by pressing the lever on a PR schedule as described above or obtaining lab chow being freely available in a dish (about 15 g) within the operant chamber. The food receptacle and the lever were positioned on the same wall of the operant chamber, the dish containing the lab chow was situated in a corner on the opposite side of the operant chamber. A session lasted for 90 min or ended when a rat failed to press the lever for consecutive 10 min: Thereafter the amount of lab chow ingested was calculated. Animals of all treatment groups received vehicle/drug infusions 30 min pre-test.

### Test 3: Consumption test

The animals were placed individually in separate cages (type **III)** containing a glass bowls filled with 30 g of pellets. The animals had free access to the reward for 20 min and the amount consumed was measured by subtracting the weight of each glass bowl before the 20 min consumption test and the weight of each glass bowl after the test. Animals of all treatment groups received vehicle/drug infusions 30 min pre-test.

### Statistical Analysis

The data were subjected to one or two way ANOVAs followed by Dunnett's post hoc test. All statistical computations were carried out with STATISTICA™ (StatSoft®, Tulsa, USA). The level of statistical significance (α-level) was set at p < 0.05.

### Results

Present data show that (R)-phenylpiracetam increases motivation, *i.e.,* the work load, which animals are willing to perform to obtain more rewarding food. At the same time consumption of freely available normal food does not increase. Generally this indicates that (R)-phenylpiracetam increase motivation and in turn positive effect on excessive sleepiness would be expected. The effect of (R)-phenylpiracetam is much stronger than that of methylphenidate and amphetamine. Moreover, (S)-phenylpiracetam produces only a very weak effect at 100 mg/kg, which is 2.5 times lower than (R)-phenylpiracetam, and no significant effect at 200 mg/kg.

### Example 7: Treatment of EDS in Parkinson Patients with Phenylpiracetam

In a prospective, randomized, double-blind, placebo-controlled, parallel group, multicenter study the tolerability, safety and efficacy of (R)-Phenylpiracetam after oral administration adjunct to L-Dopa treatment is confirmed. After a screening period of one week 36 subjects with Parkinson's Disease receive 50 mg (R)-Phenylpiracetam or placebo bid at 8.00 am and 3.00 pm for 4 weeks. A safety follow-up is performed 1 week after the double-blind treatment period. Visits take place 1 week before randomization (V1, day -7), at baseline (V2, day 1), at Week 2 (V3) and Week 4 (V4). A safety follow-up (V5) is performed 1 week after the last intake of study medication. An assessment of excessive daily sleepiness using the Epworth Sleepiness Scale [ESS] takes place at screening, baseline, week 2 and week 4.

The questionnaire asks the subject to rate his or her probability of falling asleep on a scale of increasing probability from 0 to 3 for 8 different situations that most people engage in during their daily lives, though not necessarily every day (Johns et al. J Neurol Neurosurg Psychiatry. 2005;76(12):1636). The scores for the 8 questions are added together to obtain a single number. A number in the 0-9 range is considered to be normal while a number in the 10-24 range indicates that expert medical advice should be sought. For instance, scores of 11-15 are shown to indicate the possibility of mild to moderate sleep apnoea, where a score of 16 and above indicates the possibility of severe sleep apnoea or narcolepsy.

The absolute change of ESS from day 1 to day 14 and 28 is analyzed. An improvement of at least 3 points is regarded as clinically significant and is demonstrated in this study.

## Claims

1. A pharmaceutical composition comprising (R)-phenylpiracetam or a pharmaceutically acceptable salt thereof for use in the treatment of a sleep disorder.

2. The pharmaceutical composition of claim 1, wherein said sleep disorder is excessive daytime sleepiness, particularly excessive daytime sleepiness in narcolepsy or in Parkinson's Disease.

3. The pharmaceutical composition of claim 1, wherein said sleep disorder is narcolepsy.

4. The pharmaceutical composition of claim 1, wherein said sleep disorder is excessive daytime sleepiness in patients with Parkinson's Disease.

5. The pharmaceutical composition of claim 4, wherein said sleep disorder is not a sleep disorder caused by D2-dopamine receptor agonists in the treatment of Parkinson's Disease.

6. The pharmaceutical composition of claim 4, wherein said sleep disorder is not a sleep disorder caused by agents for dopamine-replacement therapy in Parkinson's Disease.

7. The pharmaceutical composition of any one of claims 1 to 3, wherein said sleep disorder is not a sleep disorder in patients with Parkinson's Disease.

8. The pharmaceutical composition according to any one of claims 1 to 7, wherein (R)-phenylpiracetam is present as (R)-phenylpiracetam hydrochloride.

9. The pharmaceutical composition according to any one of claims 1 to 8, wherein (R)-phenylpiracetam is for administration in a range from about 1 mg to about 250 mg/day, or in a range from about 25 mg to about 200 mg/day, or in a range from about 50 mg to about 150 mg/day.

10. The pharmaceutical composition according to any of the preceding claims, wherein (R)-phenylpiracetam or a pharmaceutically acceptable salt thereof is for administration once a day, twice a day, or three times a day.

11. The pharmaceutical composition according to any of the preceding claims, wherein (R)-phenylpiracetam or a pharmaceutically acceptable salt thereof is for administration in an oral formulation.

12. The pharmaceutical composition according to any of the preceding claims, wherein (R)-phenylpiracetam or a pharmaceutically acceptable salt thereof is for administration in combination with at least one additional pharmaceutical agent which has been shown to be effective for the treatment of said sleep disorder.
